# NOUVEAU FASCICULE DE BREVET EUROPEEN

(11) **EP 0 724 642 B2**
(45) Date de publication et mention de la décision concernant l'opposition: **21.07.2010**
(45) Mention de la délivrance du brevet: 09.08.2006
(21) Numéro de dépôt: 94914452.1
(22) Date de dépôt: 27.04.1994
(51) Int. Cl.: C12N 15/85, A61K 48/00, A61K 39/12, A61K 39/29

(54) **VECTEUR NUCLEOTIDIQUE, COMPOSITION LE CONTENANT ET VACCIN POUR L'IMMUNISATION A L'ENCONTRE D'UNE HEPATITE**
NUKLEINSÄUREVEKTOR, EINEN SOLCHEN VEKTOR ENTHALTENE VERBINDUNG UND IMPFSTOFF ZUR IMMUNISIERUNG GEGEN HEPATITIS
NUCLEOTIDE VECTOR, COMPOSITION CONTAINING SUCH VECTOR AND VACCINE FOR IMMUNIZATION AGAINST HEPATITIS

(30) Priorité: 22.10.1993 FR 9312659
(43) Date de publication de la demande: 07.08.1996
(73) Titulaire: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR); UNIVERSITE D'OTTAWA/UNIVERSITY OF OTTAWA, Ottawa, Ontario (CA)
(72) Inventeur: DAVIS, Heather Lynn, Ottawa, Ontario (CA); WHALEN, Robert Gérald, F-75015 Paris (FR); MICHEL, Marie-Louise, F-75018 Paris (FR)
(74) Mandataire: Michelet, Alain
(86) Numéro de dépôt international: PCT/FR1994/000483
(87) Numéro de publication internationale: WO 1995/011307

(56) Documents cités:
- WO-A-88/06185
- WO-A-92/06212
- WO-A-93/09236
- WO-A-93/17111
- US-A- 4 592 742
- PROCEEDINGS OF THE NATIONAL ACADEMY OF SCIENCES OF USA., vol.90, 1993, WASHINGTON US pages 4156 - 4160 WANG ET AL. cité dans la demande
- SCIENCES ET AVENIR, Septembre 1993, FR pages 22 - 25 P.CHAMBON cité dans la demande
- DEVELOPMENT, vol.117, no.3, Mars 1993, CAMBRIDGE GB pages 947 - 959 LI Z ET AL 'DESMIN SEQUENCE ELEMENTS REGULATING SKELETAL MUSCLE-SPECIFIC EXPRESSION IN TRANSGENIC MICE'

## Description

La présente demande a pour objet un vecteur pour l'immunisation à l'encontre d'une hépatite.

Elle est en outre relative à une composition contenant ce vecteur.

L'immunisation par injection d'ADN nu dans les tissus musculaires a fait l'objet de plusieurs travaux depuis le début des années 1990.

Ainsi, ULMER et al. (Science, 259, 1745-1749, 1993) ont obtenu une protection à l'encontre du virus Influenza par induction des lymphocytes T cytotoxiques en injectant dans des quadriceps de souris un plasmide codant pour la nucléoprotéine Influenza A. Le plasmide utilisé porte soit le promoteur du virus du sarcome de Rous soit le promoteur du cytomégalo virus.

RAZ et al. (Proc. Natl. Acad. Sci. USA, 90, 4523-4527, 1993) ont injecté des vecteurs comprenant le promoteur du virus du sarcome de Rous et un gène codant pour l'interleukine-2, l'interleukine-4 ou le facteur de croissance transformant de type β1(TGF-β1). Les réponses immunitaires humorale et cellulaire, des souris auxquelles ont été administrés par voie intramusculaire ces plasmides, sont améliorées.

WANG et al. (Proc. Natl. Acad. Sci. USA, 90,4156-4160, 1993), ont injecté dans les muscles de souris un plasmide portant un gène codant pour la protéine d'enveloppe du virus HIV-1. L'injection des plasmides a été précédée par un traitement par de la bupivacaine au même endroit dans le muscle. Les auteurs mettent en évidence la présence d'anticorps capables de neutraliser l'infection par le virus HIV-1. On remarquera néanmoins que l'ADN a été injecté deux fois par semaine pour un nombre total de quatre injections.

DAVIS et al. (Compte-Rendu du 28ème Congrès Européen sur le muscle, Bielefeld, Allemagne, 21-25 Septembre 1992) ont injecté des plasmides portant un gène codant pour la luciférase ou la β-galactosidase en prétraitant les muscles par du sucrose ou une cardiotoxine. Les auteurs observent l'expression de la luciférase ou de la β-galactosidase.

Plus récemment, un article paru dans Science et Avenir (Septembre 1993, pages 22-25) indique que WHALEN et DAVIS ont réussi à immuniser des souris contre le virus de l'hépatite B en leur injectant de l'ADN pur du virus dans les muscles. Une injection préalable de toxine de venin de serpent suivie de l'injection d'ADN 5 à 10 jours plus tard est citée de manière générale. Il est précisé que cette méthode n'est pas pratique.

Ces travaux avaient été précédés par d'autres expérimentations dans lesquelles différents ADN avaient été injectés, en particulier dans des tissus musculaires. Ainsi, la demande PCT/US 90/01 515 (publiée sous le n° WO-90/11 092) divulgue diverses constructions plasmidiques pouvant être injectées, en particulier dans des tissus musculaires pour le traitement de la dystrophie musculaire. Néanmoins, ce document précise que l'ADN est préférentiellement injecté dans des liposomes.

Il en est de même du brevet canadien CA 362.966 (publié sous le n° 1.169.793) qui décrit l'injection intramusculaire de liposomes contenant de l'ADN codant en particulier pour les antigènes HBs et HBc. Les résultats décrits dans ce brevet mentionnent l'expression d'antigènes HBs. La présence d'anticorps anti-HBs n'a pas été recherchée.

La demande Internationale PCT/FR 92/00 898 (publiée sous le n° WO-93/06 223) décrit des vecteurs viraux pouvant être acheminés par la voie sanguine jusqu'à des cellules cibles. Ces vecteurs sont ainsi reconnus par les récepteurs de cellules, telles que des cellules musculaires et peuvent être employés dans le traitement de la dystrophie musculaire ou de la thrombose.

Cette demande ne concerne pas l'immunisation à l'encontre de virus tels que par exemple celui de l'hépatite B.

Il ressort donc de l'état de la technique cité que, si l'on connaissait déjà des techniques d'immunisation à l'encontre de l'hépatite par injection d'ADN nu, celles-ci présentaient un grand nombre d'inconvénients ne les rendant pas pratiques à mettre en oeuvre.

D'autre part, l'ADN nu utilisé pour vacciner des souris était l'ADN pur du virus. Ce type de traitement n'est pas envisageable pour la vaccination humaine, du fait des risques qu'il fait courir aux patients.

Enfin, les expérimentations plus anciennes dans lesquelles l'ADN injecté est contenu dans des liposomes n'ont pas démontré de réponse immunitaire.

Le demandeur s'est donc attaché à trouver de nouvelles constructions de vecteurs permettant d'immuniser à l'encontre de l'hépatite tout en n'ayant pas de conséquences négatives pour la santé humaine.

Il s'est d'autre part attaché à trouver un additif à des compositions contenant ces constructions permettant une dégénérescence efficace du tissu musculaire avant l'injection de l'ADN, et compatible avec les impératifs de la santé humaine.

Le demandeur a montré de manière surprenante que l'on pouvait obtenir un niveau d'anticorps efficace et durable largement supérieur au niveau permettant d'obtenir chez l'homme une protection immunitaire efficace et durable à l'encontre de l'infection par le virus de l'hépatite en administrant par injection intramusculaire un vecteur ayant une construction définie, et une substance capable d'induire une nécrose coagulatrice des fibres musculaires.

La présente demande a donc pour objet un vecteur nucléotidique comprenant :
(i) un gène ou un ADN complémentaire codant pour au moins une partie de la protéine HBs du virus de l'hépatite B,
(ii) la séquence de terminaison non traduite dudit gène ou dudit ADN complémentaire, incluant le signal de polyadénylation, située en aval dudit gène ou dudit ADN complémentaire,
(iii) un promoteur contrôlant l'expression dudit gène ou dudit ADN complémentaire dans des cellules musculaires, ledit promoteur étant choisi parmi :
   - le promoteur du cytomégalovirus,
   - le promoteur des gènes de surface du virus HBV, et
   - le promoteur d'un gène d'une protéine du cytosquelette.

Ledit vecteur peut ne pas se répliquer dans ces cellules .

Il peut aussi être réplicatif ce qui permet d'obtenir un nombre élevé de copies par cellule et d'améliorer la réponse immunitaire.

Le vecteur est en outre choisi afin d'éviter son intégration au sein de l'ADN cellulaire, de telles intégrations étant connues pour activer les oncogènes et induire la cancérisation des cellules.

Le vecteur selon la présente invention est avantageusement un plasmide en partie d'origine bactérienne et portant notamment une origine de réplication bactérienne et un gène permettant sa sélection, tel qu'un gène de résistance à un antibiotique.

Ce vecteur peut être aussi pourvu d'une origine de réplication lui permettant de se répliquer dans les cellules musculaires de son hôte, telle qu'une origine de réplication du virus du papillome bovin.

Le gène ou l'ADN complémentaire porté par ce vecteur code pour au moins une partie de la protéine HBs, sous l'une de ses formes S, S-préS2 ou S-préS2-préS1, auquel cas le gène est le gène S.

Les séquences des gènes ou des protéines des virus des hépatites sont décrites ou peuvent être déduites des documents suivants :
brevet FR-79 21 811, brevet FR 80.09.039, brevet EP-81.400.634, brevet FR 84.03.564, brevet EP 91.830.479 et article de Najarian et al. (Proc. Natl. Acad. Sci. USA, 1985, 82, 2627-2631).

Le vecteur peut aussi comprendre des gènes codant au moins en partie pour la protéine gp160 du virus HIV1 associée à la protéine p25, et/ou à la protéine p55, et/ou à la protéine p18 ou au moins un gène codant pour la protéine Rev du virus HIV1.

Le promoteur porté par ce vecteur est avantageusement le promoteur du cytomégalovirus (CMV). Il peut néanmoins être tout autre promoteur permettant une expression efficace du gène dans les cellules musculaires, tel que défini dans la revendication 1.

Il peut ainsi être :
- un promoteur d'un gène d'une protéine du cytosquelette, en particulier de la desmine tel que décrit par BOLMONT et al. (Journal of submicroscopic cytology and pathology, 1990, 22, 117-122) et ZHENLIN et al. (Gene, 1989, 78, 243-254).
- le promoteur des gènes de surface du virus HBV.

De manière générale , le promoteur peut être hétérologue à l'hôte, c'est-à-dire qu'il n'est pas trouvé chez l'hôte de manière naturelle, mais il est avantageusement homologue, tout en étant actif à l'origine dans un tissu autre que le tissu musculaire.

Outre le promoteur, le vecteur peut comprendre une séquence de terminaison de la transcription, située en aval du gène.

Un tel vecteur peut être le plasmide pCMV/HBS, ou pRCCMV-HBS, ayant la séquence SEQ ID N°1, déposé sous le N°I-1370 auprès de la Collection Nationale de Cultures des Microorganismes de l'Institut Pasteur (CNCM) le 21 Octobre 1993.

Il peut aussi être le plasmide pRSV/HBS déposé sous le n° I-1371 auprès de la CNCM le 21 Octobre 1993..

Ce plasmide est de structure similaire au pCMV/HBS mais comprend le promoteur du virus du Sarcome de Rous (RSV) au lieu du promoteur du cytomégalovirus (CMV).

D'autres plasmides peuvent être :
- le pCMVHB-S1.S2.S qui a été construit par insertion du fragment Bgl II-Bgl II du gène S, obtenu à partir du pCP10, dans un vecteur pBlueScript modifié pour contenir des sites de clonage supplémentaires dans la partie "polylinker". Le fragment contenant le gène S a été ensuite enlevé par digestion KpnI-BssH II puis cloné dans les sites correspondants du pcDNA 3 (In vitrogen, Rad Systems Europe Ltd, Abingdon UK) afin d'obtenir le pCMVHB-S1.S2.S. Ce plasmide a été déposé sous le n° I-1411 auprès de la CNCM.
- le pCMVHB-S2.S qui a été obtenu en éliminant la partie pré-S1 du gène HBS du pCMVHB-S1.S2.S par digestion KpnI/MstI, puis en liguant, après traitement par de la nucléase S1, les deux extrémités.
   Le pCMVHB-S2.S a été déposé auprès de la CNCM sous le n°I-1410.
- le pHBV-S1.S2.S, déposé auprès de la CNCM sous le n° I-1409, a été obtenu par insertion du fragment Bgl II- Bgl II du gène S, obtenu à partir du pCP10, dans un vecteur pBlueScript modifié pour contenir des sites supplémentaires de clonage dans la partie "polylinker",

La présente invention est en outre relative à des vecteurs nucléotidiques tels que décrits précédemment comprenant un promoteur homologue à l'hôte et une autre séquence de régulation pour l'expression d'un gène ou d'un ADN complémentaire codant pour une des protéines citées ci-dessus.

La présente invention a en outre pour objet un vaccin ou un médicament contenant au moins un vecteur, ou une séquence nucléotidique, tel que défini ci-dessus.

On décrit de plus une composition capable d'induire une réponse cytotoxique constituée par au moins une séquence nucléotidique exprimée dans les cellules musculaires et comportant un promoteur tel que défini ci-dessus.

Elle est encore relative à une composition pharmaceutique non lipidique destinée à l'immunisation à l'encontre d'une infection virale telle qu'une hépatite comprenant au moins d'une part une substance capable d'induire une nécrose coagulatrice des fibres musculaires et d'autre part un vecteur tel que décrit ci-dessus ou comportant une des séquences nucléotidiques telles que décrites ci-dessus complètes ou partielles. On entend par séquence partielle une séquence codant pour au moins 6 acides aminés.

Avantageusement ladite substance est la bupivacaine.

D'une manière avantageuse, ladite composition est caractérisée en ce que le vecteur est administré dans le muscle de l'individu à immuniser, au minimum 5 jours après l'administration de la bupivacaine, et sensiblement au même endroit.

Une telle pré-administration de bupivacaine permet de manière surprenante d'augmenter l'efficacité de l'administration du vecteur ainsi que l'immunisation de l'individu .

Avantageusement, le vecteur est administré dix jours après administration de la bupivacaine, et sensiblement au même endroit dans le muscle de l'individu.

La présente composition peut en outre contenir des adjuvants compatibles et pharmaceutiquement acceptables.

Une telle composition est préférentiellement administrée par injection intramusculaire. L'injection peut être effectuée à l'aide d'une seringue affectée à cet usage mais aussi à l'aide d'un pistolet à jet liquide tel que celui décrit par FURTH et al. (1992, Anal. Biochem.205, 365-368).

Les quantités de bupivacaine nécessaires pour obtenir une dégénérescence suffisante du tissu musculaire afin d'obtenir une immunisation optimale sont de l'ordre de 0,10 mg à 10mg par dose de composition injectable.

Les quantités de vecteurs à injecter afin d'obtenir une immunisation optimale de l'individu à l'encontre d'une hépatite varient en fonction de la protéine codée par le gène porté par le vecteur. A titre indicatif, on injectera entre 0,1 et 1000 µg de vecteurs par individu.

Les vecteurs pourront être obtenus par les méthodes connues de l'homme du métier, en particulier par synthèse ou par les méthodes du génie génétique.

De telles méthodes sont celles décrites en particulier dans le manuel technique :
Maniatis T. et al. 1982 - Molecular Cloning, A Laboratory Manual, Cold Spring Harbor - Ed. New York.

La présente invention est illustrée sans aucunement être limitée par les exemples qui suivent dans lesquels :
La figure 1 est une représentation schématique du plasmide pRC/CMV-HBs.
Les figures 2A à 2D sont des représentations schématiques respectivement des plasmides pCMVHB-S, pCMVHB-S2.S, pCMVHB-S1.S2.S et pHBV-S1.S2.S

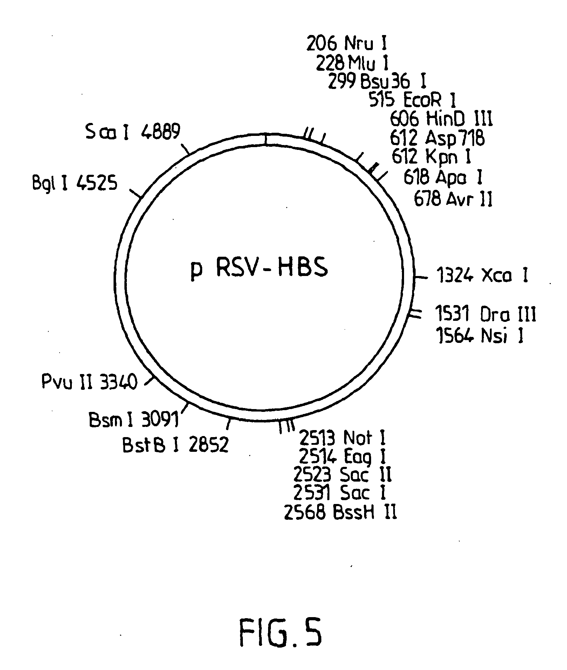

Les figures 3, 4 et 5 sont des cartes de restriction schématiques respectivement des plasmides pCMVHB-S2.S, pCMVHB-S1.S2.S et pRSV-HBS.
La figure 6 illustre la secrétion de particules antigéniques HBs (HBs Ag) en ng/ml (en ordonnée) en fonction du nombre de jours (en abscisse) par des cellules portant les plasmides pCMVHB-S, pCMVHB-S1.S2.S, pHBV-S1.S2.S, pSVS ou pCMVHB-S2.S
Les figures 7A et 7B illustrent la mise en évidence sur certaines particules de la figure 6 des antigènes prés₁ et PréS₂ par respectivement des anticorps anti-préS₁ et anti-préS2. La formation des complexes anticorps-antigène est mise en évidence par la densité optique (en ordonnée), en fonction de la concentration en antigène.
Les figures 8A à 8D représentent les réponses anti-HBS (HBs Ab en ordonnée, exprimée en mUI/ml) et anti-préS2 (preS2 Ab en ordonnée, exprimée en D.O.) de souris vaccinées par respectivement le pCMVHB-S (8A), le pCMVHB-S2.S (8B), le pCMVHB-S1.S2.S (8C) et le pHBV-S1.S2.S (8D).
La figure 9 illustre la réponse anticorps, immunoglobulines Ig G et IgM (titres en ordonnée), d'une souris vaccinée par le pCMVHB-S2.S en fonction du nombre de semaines (en abscisse).
Les figures 10A à 10C représentent les réponses anti-groupe et anti-sous type ay induites par de l'ADN de pCMV-S (ADN) ou de l'antigène HBS (prot), respectivement chez des souris B10 (10A), B10S (10B) et B10M (10C).
Les figures 10D à 10F représentent les réponses anti-groupe a induites par de l'ADN de pCMV-S (ADN) ou de l'antigène HBS (prot), respectivement chez des souris B10 (10D), BIOS (10E) et B10M (10F).

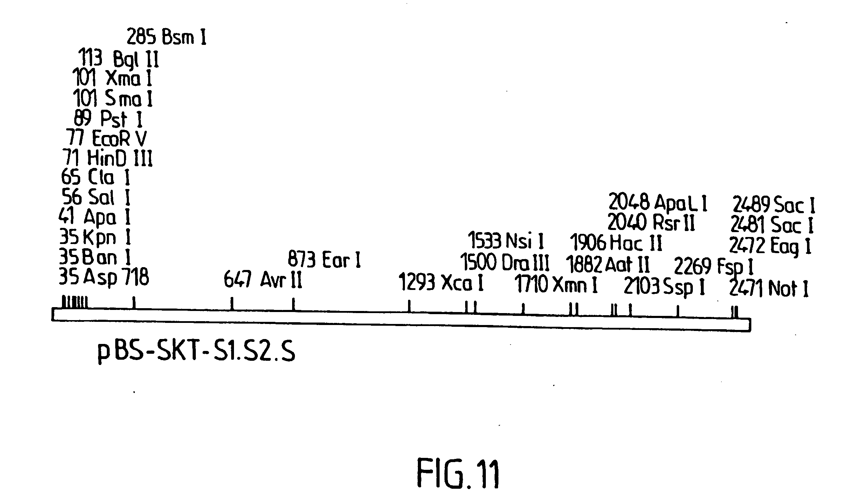

La figure 11 représente une carte linéaire de restriction du plasmide pBS-SKT-S1.S2.S.

### EXEMPLE 1 :

### Induction d'anticorps à l'encontre d'un antigène de surface de l'hépatite B par injection séquentielle de bupivacaine et d'un plasmide portant un gène codant pour l'antigène.

### 1) Matériels et méthodes

### 1.1 Prétraitement par la bupivacaine.

Toutes les expérimentations ont été effectuées sur les muscles du tibia antérieur (TA) de souris C57BL/6J âgées de 5 à 7 semaines.

Un cycle unique de dégénération et de régénération des fibres musculaires est induit dans les muscles du tibia antérieur de souris non anesthésiées, par injection intramusculaire de 50 µl de marcaïne (bupivacaine 0,5 %, DMSO 1%) commercialisée par les Laboratoires Astra, France. La solution est injectée en utilisant une seringue à tuberculose et une aiguille insérée dans un manchon de polyéthylène, afin de limiter la pénétration à une profondeur de 2 mm.

La marcaïne étant un anesthésique, les injections dans les jambes droite et gauche sont espacées de 10 à 30 minutes afin d'éviter un surdosage.

### 1.2. Préparation de l'ADN

Le plasmide utilisé a été construit par clonage dans un vecteur pBluescript modifié du fragment de restriction Xho I-Bgl II du plasmide pCP10 qui contient le gène codant pour l'antigène de surface HBS et des séquences non traduites en amont et en aval incluant le signal de poly-adénylation.

Le gène S a été ensuite récupéré par digestion à l'aide des enzymes KpnI-BssHII et le fragment a été cloné dans le site du vecteur pRC/CMV commercialisé par In Vitrogen. La construction plasmidique finale a été appelée pCMV-HBS et a été déposé sous le n° I-1370 auprès de la CNCM.

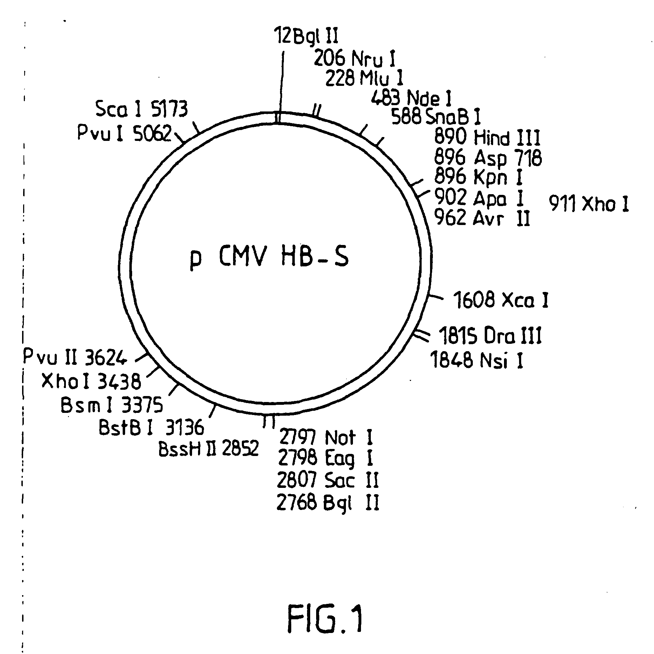

Ce plasmide est représenté schématiquement sur la figure 1. Le promoteur CMV est situé entre le nucléotide 228 qui est le site de coupure de la MluI et le nucléotide 896, qui est le site de coupure de la KpnI. Le fragment d'ADN comprenant le gène de structure de l'antigène HBs a été cloné entre les nucléotides 896 et 2852 (site de la BssH III).

Le gène HBs s'étend entre les nucléotides 911 (site XhoI) et 2768 (site Bgl II).

La séquence complète de ce plasmide est la séquence SEQ ID N°1.

L'ADN plasmidique purifié a été préparé par les méthodes standard, redissous dans du tampon PBS et stocké à -20°C jusqu'à l'injection.

### 1.3 Injection de l'ADN

Un à cinq jours après injection de la marcaïne, on a injecté au même endroit l'ADN, la souris étant anesthésiée à l'aide de pentobarbital de sodium (75mg/kg voie intrapéritonéale).

La solution d'ADN qui contient 50 µg d'ADN plasmidique et 50 µl de tampon PBS a été injectée à travers la peau dans les muscles en cours de régénération du tibia antérieur par une injection unique intramusculaire.

Des injections ont été effectuées de manière bilatérale, dans les deux pattes des souris, chaque animal recevant ainsi au total 100µg d'ADN plasmidique recombinant. Comme pour l'injection de la marcaïne, on injecte la solution d'ADN en utilisant la seringue à tuberculose et l'aiguille précédemment décrite.

Dans chaque patte on a effectué une seule injection intramusculaire d'ADN.

### 2. Résultats

Les résultats obtenus sont résumés dans le tableau I ci-après .

Ils montrent de manière très claire que l'injection d'ADN après traitement par la marcaine permet d'obtenir des taux importants d'anticorps sériques à l'encontre de l'antigène de surface de l'hépatite B.

Ces résultats sont surprenants, l'analyse de l'état de la technique ne permettant pas de supposer qu'un plasmide permettrait l'induction d'anticorps-anti-HBs pouvant être retrouvés dans le sérum et permettant ainsi une vaccination efficace.

La facilité de mise en oeuvre de la vaccination par des plasmides, et le fait qu'il ne soit pas nécessaire de faire des rappels, permet d'envisager une vaccination à grande échelle.

### EXEMPLE 2 :

### Comparaison de l'efficacité d'injection d'un plasmide en présence et en absence de lipides.

Une dose de 10 µg d'ADN plasmidique du vecteur SV40-Luciférase disponible dans le commerce' ("pGL2-Control Vector" de Promega, référence El 11) dans 50 µl de solution physiologique est injectée dans un muscle prétraité avec du sucrose selon la méthode de Davis et al. (Hum. Gene Ther. 4:151-159 (1993)). L'ADN injecté est préalablement mélangé avec des lipides tels que le dioctadécylamidoglycyl spermine (DOGS) ou des mélanges suivants : DOGS + spermidine, et DOGS + polyéthylèneglycol (PEG). L'activité de la luciférase est mesurée 5 jours après l'injection.

Ces résultats figurent dans le tableau II ci-après.

Ils montrent que la présence de lipides (DOGS) diminue de manière très importante l'efficacité d'injection du plasmide par rapport à une composition sans lipides (témoin).

### EXEMPLE 3:

### Comparaison des réponses de souris et de lapins à l'encontre de plasmides portant divers promoteurs et gènes d'enveloppe du virus HBV.

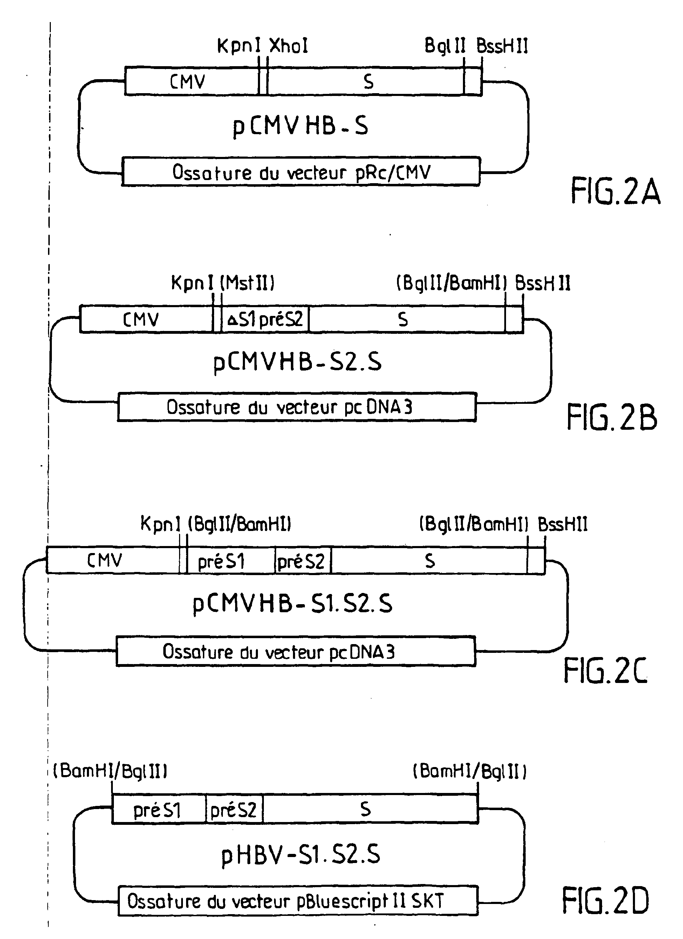

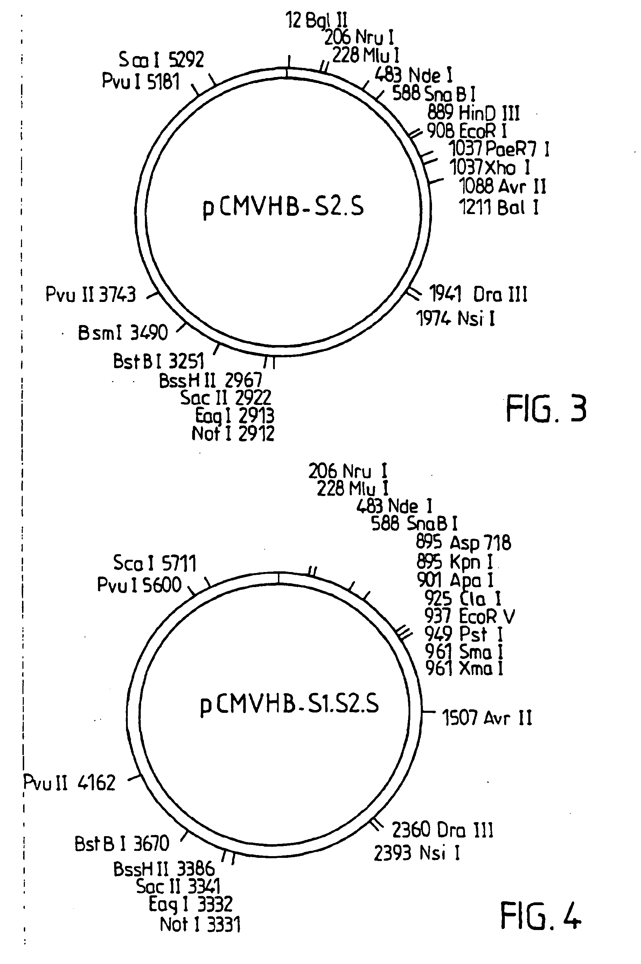

Quatre plasmides ont été construits permettant l'expression de une, deux ou trois protéines d'enveloppe du virus HBV. Dans trois des constructions (pCMVHB-S, pCMVHB-S2.S, pCMVHB-S1.S2.S) les gènes codant pour les protéines d'enveloppe du virus HBV sont placés sous le contrôle transcriptionnel du promoteur des gènes précoces du virus CMV (figure 1, figure 2A à 2C , figures 3 et 4). Le quatrième plasmide (pHBV-S1.S2.S) utilise comme élément de contrôle transcriptionnel le promoteur des gènes de surface du virus HBV contenu dans la région pré-S1 de ce virus (Cattaneo et al. (1983) Nature, 305, 336) (figure 2D). Dans les quatre constructions, le signal de polyadénylation utilisé est contenu dans les séquences HBV présentes en 3' du gène S.

### 1. Contrôle in vitro de l'efficacité des vecteurs.

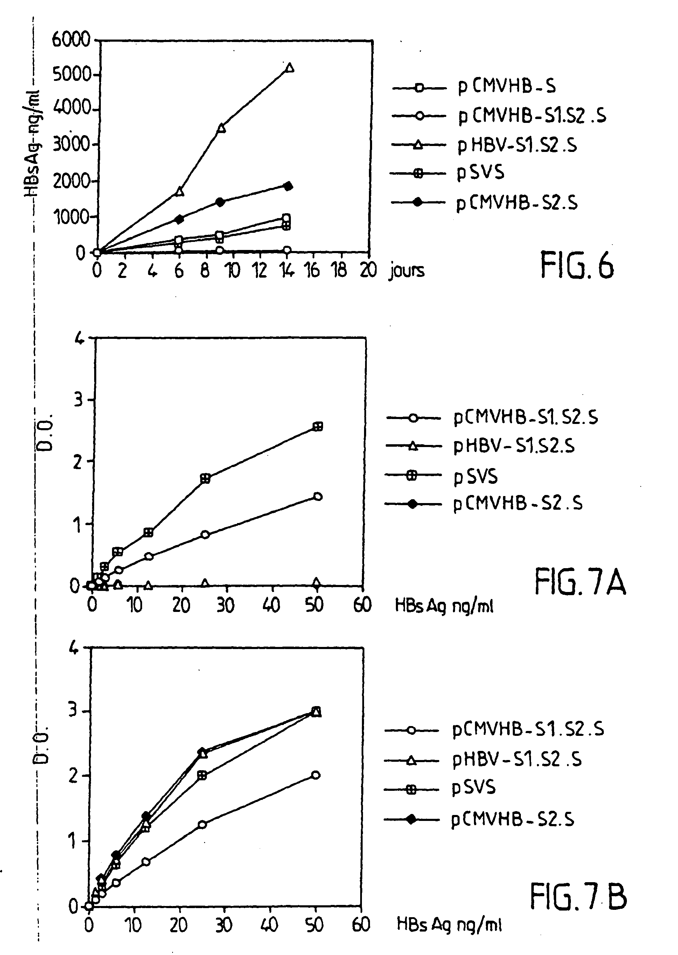

Pour contrôler l'efficacité de ces vecteurs in vitro dans des cellules eucaryotes, des fibroblastes ou des myoblastes de souris ont été transfectés. Un plasmide exprimant les trois protéines d'enveloppe sous contrôle du promoteur SV40 (pSVS) a été utilisé comme contrôle (Michel et al. 1984, Proc. Natl. Acad. Sci. USA, 81, 7708-7712)). La figure 6 montre la cinétique de sécrétion des particules HBs dans les surnageants de culture. Les faibles taux d'antigène produits par transfection du vecteur pCMVHB-S1.S2.S sont compatibles avec une forte synthèse de la grande protéine d'enveloppe à partir du promoteur CMV. Cette protéine étant myristillée dans sa partie amino-terminale, est retenue dans le réticulum endoplasmique (Ganem, (1991), Current Topics in Microbiology and Immunology, 168, 61-83). La rétention dans la cellule de protéines portant des déterminants pré-S1 a été confirmée par immunofluorescence.

La composition des particules sécrétées a été analysées dans un système ELISA sandwich utilisant comme anticorps de capture un anticorps monoclonal de souris spécifique des déterminants pré-S1 (figure 7A) ou pré-S2 (figure 7B) et comme second anticorps un sérum polyclonal de lapin anti-HBs. Ces expériences montrent que les particules d'AgHBs produites à partir du vecteur pCMVHB-S1.S2.S portent des déterminants pré-S1 et pré-S2 signant la présence de la grande et de la moyenne protéine d'enveloppe du virus HBV. Les particules sécrétées après transfection du vecteur pCMVHB-S2.S et pHBV-S1.S2.S portent, en plus des déterminants Hbs, des déterminants pré-S2 caractéristiques de la protéine moyenne d'enveloppe.

### 2) Inoculation de l'ADN

L'ADN purifié sur colonne Quiagen a été injecté par voie intramusculaire en une seule injection de 100µg (50 µg/patte) dans le muscle antérieur du tibia de souris C57/BL6 (8 souris par groupe). Cinq jours avant l'injection, le muscle a été prétraité par la cardiotoxine afin d'induire une dégénération suivie d'une régénération des cellules musculaires favorisant ainsi la capture de l'ADN par ces cellules.

Des expériences d'injection de l'ADN ont été également réalisées chez le lapin. Dans ce cas l'ADN pCMVHB-S a été administré dans le muscle normal sans dégénération, soit au moyen d'un pistolet d'injection sans aiguille le Biojector^{R} , soit au moyen de seringues conventionnelles munies d'aiguilles.

### 3) Réponses anti-HBs des souris vaccinées par l'ADN

Une réponse anticorps anti-HBs est induite par une seule injection de l'un ou l'autre des quatre plasmides utilisés.

La réponse anticorps a été analysée au moyen de kit commerciaux de détection des anticorps anti-HBs (Monolisa anti-HBs, Diagnostic Pasteur). Les anticorps anti-préS2 sont détectés dans un système ELISA utilisant sur la phase solide un peptide de la région pré-S2 (AA 120-145) correspondant à l'épitope B majeur porté par ce domaine (Neurath et al., (1985), Nature, 315, 154).

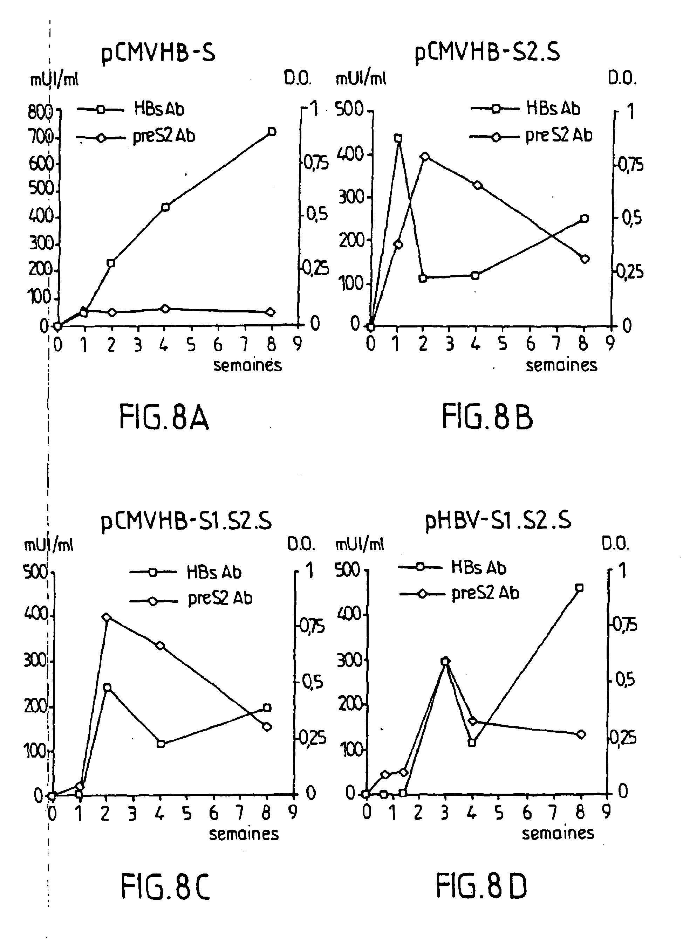

Les figures 8A à 8D montrent la cinétique de la réponse anti-HBs (HBs-Ab) exprimée en milli-unités internationales/ml et de la réponse anti-pré-S2 (préS2Ab) mesurée en densité optique (492 nm) pour des sérums dilués au 1/100. La révélation est effectuée à l'aide d'un anticorps anti-immunoglobuline (IgG) de souris marqué à la péroxydase.

L'injection du plasmide pCMVHB-S (figure 8A) induit une synthèse constante des anticorps anti-HBs. La séroconversion est obtenue chez 100% des souris dès une semaine après l'injection avec un taux moyen d'anticorps de 48 mUI/ml (de 12 à 84 mUI/ml, écart-type (SD) =28), ce qui est 4 à 5 fois supérieur au seuil requis chez l'homme pour conférer la protection (10 mUI/ml).

La réponse induite par une seule injection du plasmide pCMVHB-S2.S (figure 8B) se caractérise par l'apparition très précoce d'anticorps anti-HBs. Ces anticorps atteignent un taux moyen de 439 mUI/ml (de 104 à 835 mUI/ml; SD = 227) à une semaine puis diminuent pour réaugmenter et atteindre à 13 semaines le taux initial. La signification de ce pic d'anticorps sera discutée plus loin. Un pic d'anticorps IgG anti-pré-S2 est observé à deux semaines.

L'apparition des anticorps anti-HBs induits par l'injection des plasmides pCMVHB-S1.S2.S (figure 8C) et pHBV-S1.S2.S (figure 8D) est légèrement retardée puisque les souris ne séroconvertissent à 100 % qu'à partir de deux semaines. Le profil de la séroconversion est identique, il est caractérisé par une réponse initiale spécifique de l'antigène pré-S2 suivie d'une réponse anti-HBs augmentant graduellement pour atteindre des taux de 488 mUI/ml (de 91 à 1034 mUI/ml; SD=552) (pCMVHB-S1.S2.S) et 1725 mUI/ml (de 143 à 6037 mUI/ml; SD=1808) (pHBV-S1.S2.S) à 13 semaines.

### 4) Réponse anti-HBs des lapins vaccinés par l'ADN.

Les résultats présentés sur les tableaux III et IV montrent que les taux d'anticorps détectés à 8 semaines chez les lapins immunisés au Biojector sont significativement supérieurs à ceux obtenus par injection de l'ADN à l'aiguille.

### 5. Analyse qualitative de la réponse humorale.

Des systèmes ELISA portant sur la phase solide des antigènes HBs de compositions variées vis-à-vis des déterminants présentés et utilisant comme second anticorps des anticorps spécifiques des IgM ou des IgG de souris ont permis d'analyser qualitativement la réponse anticorps obtenue.

Dans tous les cas, l'injection unique de l'ADN chez la souris se caractérise par l'apparition précoce d'IgM spécifiques de l'AgHBs suivie immédiatement par la conversion en anticorps d'isotypes IgG caractéristiques de la réponse mémoire induite avec l'aide des cellules T auxiliaires. La réponse anticorps à l'injection de l'ADN se caractérise par sa précocité. En effet, la séroconversion est obtenue 8 à 15 jours après l'injection selon le type d'ADN utilisé et dans tous les cas le plateau est atteint en quatre semaines et maintenu de façon soutenue pendant 12 semaines.

L'utilisation d'antigène HBs de sous type hétérologue (ad) fixé sur les plaques ELISA permet de mettre en évidence la présence, dans le sérum des souris immunisées, d'anticorps spécifiques de groupe anti-a, et par différence de réactivité vis-à-vis d'AgHBs de même sous-type (ay), d'anticorps spécifiques de sous-type anti-y. La présence des anticorps spécifiques des déterminants de groupe de l'AgHBs est très importante puisque ceux-ci sont capables de conférer la protection contre des virus de sous-type hétérologues lors d'épreuves virulentes chez le chimpanzé (Szmuness et al. (1982) N. Engl. J. Med. 307, 1481-1486).

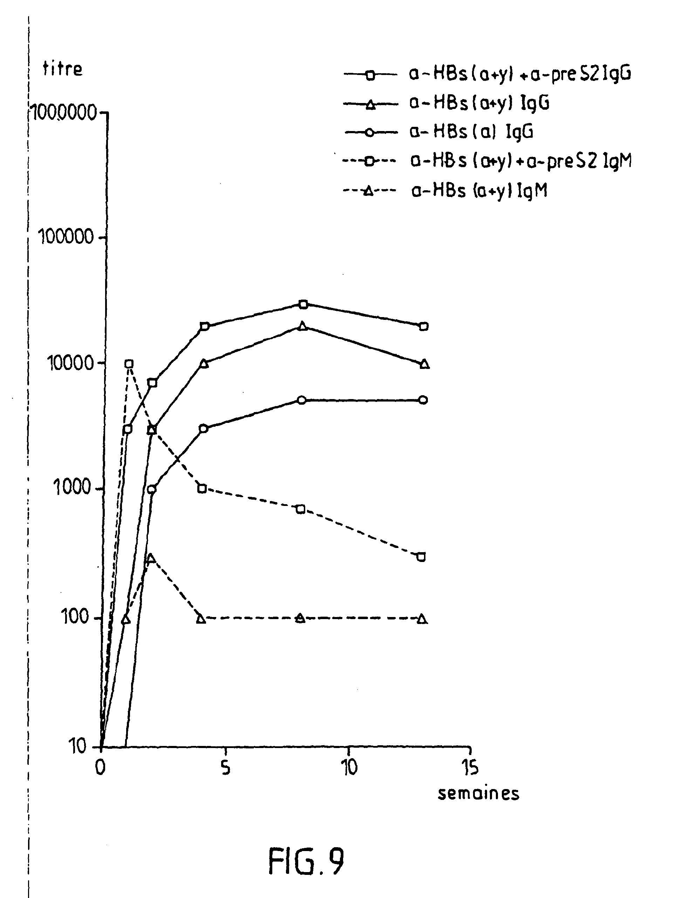

L'analyse de la réponse induite par le vecteur pCMV-S2.S montre que celle-ci présente une remarquable similitude avec celle que l'on peut observer chez l'homme au cours de l'infection. Elle est caractérisée par un pic d'IgM spécifiques de la région pré-S2 extrêmement précoce (8 jours) immédiatement suivi par une conversion en IgG anti-pré-S2 (figure 9). Cette réponse est suivie par l'apparition des anticorps anti-HBs IgM puis IgG. La production des anticorps anti-HBs est constante et atteint un maximum à 4 semaines. A 13 semaines des IgG anti-HBs et anti-pré-S2 persistent à un niveau constant.

La réponse anti-sous-type (y) précède la réponse anti-groupe (a) de la même manière que cela a été décrit lors de la vaccination avec le vaccin recombinant (Tron et al., (1989) (J. Infect. Dis.160, 199-204).

La réponse obtenue avec les trois autres ADN vaccin montre la commutation de classe IgM→IgG caractéristique de la réponse secondaire. La réponse étant d'abord dirigée contre le sous-type avant de l'être contre les déterminants de groupe de l'AgHBs.

La réponse à long terme, étudiée pour l'ADN pCMVHB-S, montre que le pic d'anticorps est atteint en 3 mois et que ceux-ci persistent à taux égal 6 mois plus tard (Tableau V).

### 6) Vaccin génétique et non réponse

Un problème majeur de la vaccination contre l'hépatite B reste le nombre élevé de non-répondeurs à la vaccination classique (2,5 à 5%) . La non réponse chez l'homme a pu être corrélée à certains types HLA (Krustall et al., (1992) J. Exp. Med. 175, 495-502) et à un défaut de la présentation de l'antigène ou de la stimulation des cellules T auxiliaires.

Pour étudier l'impact possible de la vaccination génétique sur la non réponse à l'AgHBs, un panel de souches de souris dont la réponse aux différentes protéines d'enveloppe du virus HBV est régulée génétiquement et a été bien caractérisée par Millich et Coll. (1986 J. Immunol. 137, 315) a été utilisé. La construction pCMVHB-S précédemment décrite a été injectée dans les muscles des souris B10(H-2^{b})B10.S (H-2^{S}) et B10.M (H-2^{f}).

La souche B10 répond aux trois protéines d'enveloppe du virus, la souche B10.S ne répond pas à l'AgHBs mais la non-réponse peut être contournée par immunisation avec des antigènes HBsAg portant des déterminants pré-S2. La souche B10M est totalement non répondeuse à l'antigène HBs et à l'antigène pré-S2. Une réponse dans cette souche a pu être obtenue par immunisation avec des AgHBs portant des déterminants pré-S1.

Les souris immunisées par l'ADN ont reçu une seule injection (100 µg) dans le muscle en régénération. Les souris témoins ont reçu la protéine en deux injections intrapéritonéales à un mois d'intervalle, la première de 2 µg AgHBs adjuvé en adjuvant complet de Freund (CFA) et la seconde de 2 µg AgHBs adjuvé en adjuvant incomplet de Freund (IFA).

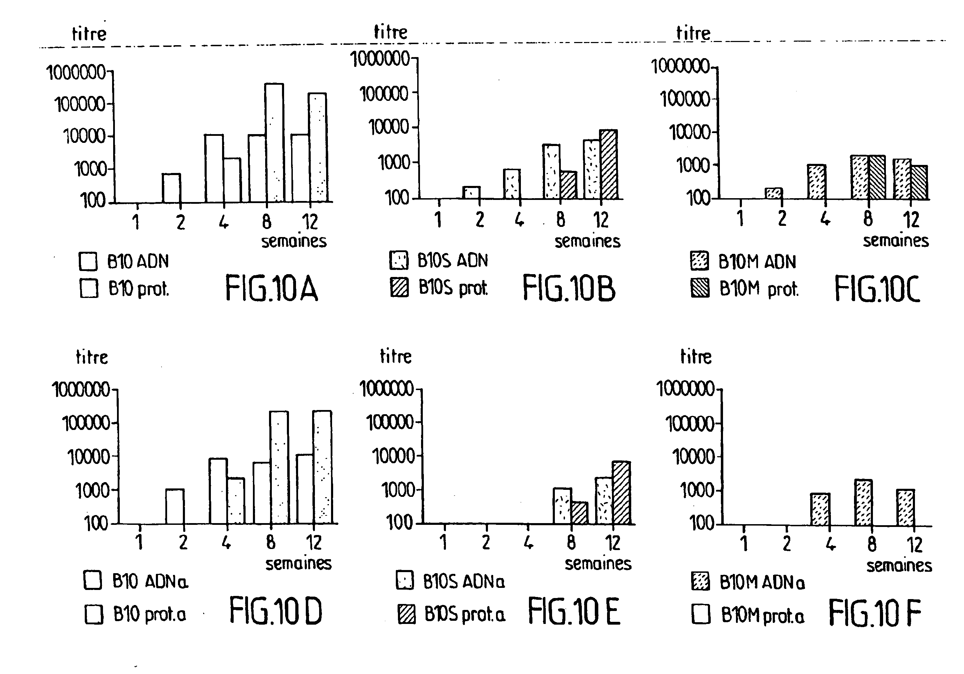

Les résultats obtenus avec l'ADN pCMVHB-S sont illustrés par les figures 10A à 10F.
- Dans la souche B10 (bonne répondeuse) la réponse induite par l'ADN est plus précoce que la réponse induite par la protéine après une seule injection.
- Dans la souche B10S (non répondeuse à l'AgHBs en l'absence de pré-S2) on observe l'apparition d'anticorps anti-HBs spécifiques de sous-type puis spécifiques de groupe après immunisation par l'ADN pCMVHB-S. Des anticorps anti-HBs spécifiques de groupe ne sont obtenus chez les souris immunisées avec la protéine HBs qu'après la 2ème injection.
- Dans la souche B10M (non répondeuse à l'AgHBs en l'absence de pré-S1) l'immunisation par l'ADN permet d'obtenir une réponse anti-HBs spécifique de groupe et de sous-type alors que la protéine induit une réponse de sous-type seulement et nécessite deux injections.

La réponse induite par les trois types de vecteurs est comparée dans les trois souches de souris.

### CONCLUSION

Il est généralement admis que la réponse humorale à l'antigène HBs est suffisante à elle seule pour conférer la protection. La présence d'anticorps dirigés contre d'autres déterminants (pré-Sl et pré-S2) portés par les protéines d'enveloppe du virus, eux-mêmes protecteurs, pourrait améliorer la qualité de cette réponse. L'ensemble des expériences rapportées ici montre que la réponse humorale induite par la vaccination génétique anti-hépatite B est dans plusieurs domaines supérieure à celle qui peut être obtenue par la vaccination classique.

En termes de taux de séroconversion: le taux de 100% est obtenu dès 8 jours pour les souris immunisées avec les ADN pCMV-HBs et pCMVHB-S2.S et cela après une seule injection.

En termes de niveau de réponse: le seuil de 10 mUI/ML considéré suffisant chez l'homme pour conférer la protection est toujours largement dépassé.

En termes de précocité de la réponse: le vecteur pCMVHB-S2.S permet d'obtenir en 8 jours un niveau très élevé d'anticorps anti-pré-S2 et l'on sait que ceux-ci sont capables, à eux seuls, de conférer la protection (Itoh et al., (1986) Proc. Natl. Acad. Sci. USA 83, 9174-9178).

En termes de persistance de la réponse: les anticorps anti-HBs persistent à un niveau élevé pendant plus de 6 mois.

En, termes de qualité de la réponse: les anticorps obtenus sont de type IgG caractéristiques d'une réponse dépendante de cellules T auxiliaires et donc d'une réponse mémoire.

En termes d'activité anti-virale: les anticorps sont spécifiques du sous-type viral mais surtout spécifiques de groupe et donc susceptibles de conférer une protection croisée.

En termes de signification biologique: le profil de la réponse obtenue par immunisation avec pCMVHB-S2.S mime complètement celui que l'on peut observer, chez l'homme, lors d'une infection virale résolue.

**TABLEAU I**

| Induction d'anticorps à l'encontre de l'antigène de surface de l'hépatite B. | | | | |
|---|---|---|---|---|
| Description | Nombre de souris | Taux d'anticorps à l'encontre de l'antigène de surface de l'hépatite B dans le sérum (mIU/ml) | | |
| | | avant l'injection d'ADN | 15 jours après l'injection d'ADN | 35 jours après l'injection d'ADN |
| ADN injecté 1 jour après le traitement par la marcaine | 5 | 0 | moyenne: 56 de 5 à plus de 140 | moyenne: 59 |
| ADN injecté 5 jours après le traitement par la marcaine | 5 | 0 | moyenne : 71 de 21 à plus de 108 | moyenne : 47 |

**TABLEAU II**

| Groupe | RLU/sec/muscle de Luciférase (Moyenne ± SEM) RLU = Unité de Lumière Relative | Pourcentage par rapport au témoin |
|---|---|---|
| Témoin | 43 082 ± 5 419 | 100 % |
| 4X DOGS | 28 ± 7 | 0,06 % |
| DOGS -Spermidine | 50 ± 23 | 0,12 % |
| PEG-DOGS | 0 ± 0 | 0,00 % |

=

**TABLEAU III**

| Immunisation au Biojector^{R} | | | |
|---|---|---|---|
| pCMV-HB.S | | | |
| N° | 0 semaine | 2 semaines | 8 semaines |
| 2.1 | 0 | 517 | 380 |
| 2.2 | 0 | 374 | 322 |
| 3.1 | 0 | 258 | 418 |
| 4.1 | 0 | 400 | 4045 |
| 4.2 | 0 | 88 | 86 |
| 4.3 | 0 | 314 | 420 |
| 6.1 | 0 | 415 | 1001 |
| 6.2 | 0 | 1543 | 3517 |
| 6.3 | 0 | 1181 | 141 |
| Moyenne | 0 | 566 mUI/ml | 1148mUI/ml |
| SD | 0 | 476 | 1521 |
| SEM | 0 | 159 | 507 |
| N | 9 | 9 | 9 |
| CV | | 84% | 133% |

=

**TABLEAU IV**

| Immunisation par injection à l'aide d'une aiguille | | | |
|---|---|---|---|
| pCMV-HB.S | | | |
| N° | 0 semaine | 2 semaines | 8 semaines |
| 1.1 | 1 | 0 | 1 |
| 5.1 | 0 | 287 | 186 |
| 5.2 | 0 | 162 | 798 |
| 5.3 | 0 | 305 | 203 |
| 7.1 | 0 | 86 | 175 |
| 7.2 | 0 | 1108 | mort |
| moyenne | 0 | 325 mUI/ML | 273 mUI/ml |
| SD | 0 | 401 | 305 |
| SEM | 0 | 164 | 136 |
| N | 6 | 6 | 5 |
| CV | 245% | 124% | 112% |

=

**TABLEAU V**

| Réponse à long terme d'une souris vaccinée par le pCMVHB-S | | | | |
|---|---|---|---|---|
| | 1 mois | 2 mois | 3 mois | 6 mois |
| * Titre a-HBs en mUI/ml | 227 | 662 | 1299 | 1082 |
| * Titre a-HBs ELISA | 3,5.10⁻⁴ | 5.10⁻⁴ | 8.5.10⁻⁴ | 9.10⁻⁴ |

## Revendications

1. Vaccin comprenant :
- un vecteur nucléotidique, ledit vecteur nucléotidique comprenant :
(i) un gène ou un ADN complémentaire codant pour au moins une partie de la protéine HBs du virus de l'hépatite B,
(ii) la séquence de terminaison non traduite dudit gène ou dudit ADN complémentaire, incluant le signal de polyadénylation, située en aval dudit gène ou dudit ADN complémentaire,
(iii) un promoteur contrôlant l'expression dudit gène ou dudit ADN complémentaire dans des cellules musculaires, ledit promoteur étant choisi parmi :
- le promoteur du cytomégalovirus,
- le promoteur des gènes de surface du virus HBV,
- un promoteur d'un gène d'une protéine du cytosquelette, et
- des adjuvants compatibles et pharmaceutiquement acceptables.

2. Vaccin selon la revendication 1, **caractérisé en ce que** ledit vecteur nucléotidique ne se réplique pas dans les cellules.

3. Vaccin selon l'une des revendications 1 et 2, **caractérisé en ce que** ledit gène ou ledit ADN complémentaire code pour la protéine S, S-préS2 ou S-préS2-préS1.

4. Vaccin selon l'une des revendications 1 à 3, **caractérisé en ce que** ledit gène est le gène S.

5. Vaccin selon l'une des revendications 1 à 4, **caractérisé en ce que** ledit vecteur nucléotidique est le plasmide pCMV-HBS déposé sous le n° I-1370 auprès de la CNCM le 21 octobre 1993.

6. Vaccin selon l'une des revendications 1 à 4, **caractérisé en ce que** ledit vecteur nucléotidique est le plasmide pCMVHB-S1.S2.S déposé auprès de la CNCM sous le n° I-1411.

7. Vaccin selon l'une des revendications 1 à 4, **caractérisé en ce que** ledit vecteur nucléotidique est le plasmide pCM VHB-S2.S déposé auprès de la CNCM sous le n° I-1410.

8. Vaccin comprenant comme vecteur nucléotidique le plasmide pRSV-HBS déposé sous le n° I-1371 auprès de la CNCM le 21 octobre 1993.

9. Vaccin selon l'une des revendications 1 à 4, **caractérisé en ce que** ledit vecteur nucléotidique est le plasmide pHBV-S1.S2.S déposé auprès de la CNCM sous le n° I-1409.

10. Vaccin selon l'une des revendications 1 à 3, **caractérisé en ce que** le promoteur d'une protéine du cytosquelette est le promoteur de la desmine.

11. Composition pharmaceutique non lipidique destinée à l'immunisation à l'encontre d'une infection virale par le virus de l'hépatite comprenant un vecteur nucléotidique tel que défini dans l'une quelconque des revendications 1 à 10 et une substance capable d'induire une nécrose coagulatrice des fibres musculaires.

12. Composition selon la revendication 11, **caractérisée en ce que** ledit vecteur est administré dans le muscle de l'individu à immuniser, au minimum cinq jours après administration de bupivacaine, sensiblement au même endroit.

13. Composition selon la revendication 11, **caractérisée en ce que** le vecteur est administré dix jours après administration de la bupivacaine.

14. Composition selon l'une des revendications 12 et 13, **caractérisée en ce que** l'administration est effectuée par injection intramusculaire.

15. Composition selon la revendication 13, **caractérisée en ce que** l'injection intramusculaire est effectuée à l'aide d'un pistolet à jet liquide.

16. Vecteur nucléotidique comprenant :
(i) un gène ou un ADN complémentaire codant pour au moins une partie de la protéine HBs du virus de l'hépatite B,
(ii) la séquence de terminaison non traduite dudit gène ou dudit ADN complémentaire, incluant le signal de polyadénylation, située en aval dudit gène ou dudit ADN complémentaire,
(iii) un promoteur contrôlant l'expression dudit gène ou dudit ADN complémentaire dans des cellules musculaires, ledit promoteur étant choisi parmi :
- le promoteur du cytomégalovirus,
- le promoteur des gènes de surface du virus HBV, et
- le promoteur d'un gène d'une protéine du cytosquelette.

17. Vecteur nucléotidique selon la revendication 16, **caractérisé en ce qu'**il est le plasmide pCMV-HBS déposé sous le n° I-1370 auprès de la CNCM le 21 octobre 1993.

18. Vecteur nucléotidique selon la revendication 16, **caractérisé en ce qu'**il est le plasmide pCMVHB.S1.52.S déposé auprès de la CNCM sous le n° I.1411.

19. Vecteur nucléotidique selon la revendication 16, **caractérisé en ce qu'**il est le plasmide pCMVHB-S2.S déposé auprès de la CNCM sous le n° I-1410.

20. Vecteur nucléotidique selon la revendication 16, **caractérisé en ce qu'**il est le plasmide pHBV-S1.S2.S déposé auprès de la CNCM sous le n° I-1409.

21. Vecteur nucléotidique qui est le plasmide pRSV-HBS déposé sous le n° I-1371 auprès de la CNCM le 21 Octobre 1993.

## Claims

1. Vaccine comprising:
- a nucleotide vector, the said nucleotide vector comprising :
(i) a gene or a complementary DNA coding for at least a portion of the hepatitis B virus protein HBs,
(ii) the non-translated termination sequence of the said gene or of the said complementary DNA, including the polyadenylation signal, located downstream of the said gene or of the said complementary DNA,
(iii) a promoter controlling the expression of the said gene or that of the said complementary DNA in muscle cells, the said promoter being selected among
- the promoter of cytomegalovirus
- the promoter of virus HBV surface genes,
- a promoter of a cytoskeleton protein, and
- compatible and pharmaceutically acceptable adjuvants

2. Vaccine according to claim 1 **characterized in that** the said nucleotide vector does not replicate in the cells.

3. Vaccine according to anyone of claims 1 or 2 **characterized in that** the said gene or the said complementary DNA encodes for the protein S, S-preS2 or S-preS2-preS1.

4. Vaccine according to anyone of claims 1 to 3, **characterized in that** the said gene is the gene S.

5. Vaccine according to anyone of claims 1 to 4, **characterized in that** the said nucleotide vector is the pCMV-HBS plasmid filed under N°I-1370 at the CNCM on October 21, 1993.

6. Vaccine according to anyone of claims 1 to 4, **characterized in that** the said nucleotide vector is the pCMVHB-S1.S2.S plasmid filed at the CNCM under N°I-1411.

7. Vaccine according to anyone of claims 1 to 4, **characterized in that** the said nucleotide vector is the pCMVHB-S2.S plasmid filed at the CNCM under N°I-1410.

8. Vaccine comprising the plasmid pRSV-HBS plasmid filed under N° I-1371 at the CNCM on October 21, 1993 as nucleotide vector.

9. Vaccine according to anyone of claims 1 to 4, **characterized in that** the said nucleotide vector is the pHBV-S1-S2.S plasmid filed at the CNCM under N°I-1409

10. Vaccine according to anyone of claims 1 to 3, **characterized in that** the promoter of a cytoskeleton protein is the promoter of desmine.

11. Non-lipidic pharmaceutical composition to be used in the immunization against a viral infection by hepatitis virus comprising a nucleotide vector as defined in anyone of claims 1 to 10 and a substance able to induce a coagulative necrosis of muscle fibers.

12. Composition according to claim 11, **characterized in that** the said vector is administered in the muscle of the individual to be immunized, at least five days after the administration of bupivacaine more or less in the same area.

13. Composition according to claim11, **characterized in that** the vector is administered 10 days after the administration of bupivacaine.

14. Composition according to anyone of claims 12 and 13, **characterized in that** the administration is carried out by intramuscular injection.

15. Composition according to claim 14, **characterized in that** the intramuscular injection is carried out using a lipid jet gun.

16. Nucleotide vector comprising:
(i) a gene or a complementary DNA coding for at least a portion of the hepatitis B virus protein HBs,
(ii) the non-translated termination sequence of the said gene or of the said complementary DNA, including the polyadenylation signal, located downstream of the said gene or of the said complementary DNA,
(iii) a promoter controlling the expression of the said gene or that of the said complementary DNA in muscle cells, the said promoter being selected among
- the promoter of cytomegalovirus
- the promoter of virus HBV surface genes, and
- a promoter of a cytoskeleton protein.

17. Nucleotide vector according to claim 16, **characterized in that** the said nucleotide vector is the pCMV-HBS plasmid filed under N°I-1370 at the CNCM on October 21, 1993.

18. Nucleotide vector according to claim 16, **characterized in that** the said nucleotide vector is the pCMVHB-S1.S2.S plasmid filed at the CNCM under N°I-1411.

19. Nucleotide vector according to claim 16, **characterized in that** the said nucleotide vector is the pCMVHB-S2.S plasmid filed at the CNCM under N°I-1410.

20. Nucleotide vector according to claim 16, **characterized in that** the said nucleotide vector is the pHBV-S1-S2.S plasmid filed at the CNCM under N°I-1409.

21. Nucleotide vector according to claim 16, **characterized in that** the said nucleotide vector is the plasmid pRSV-HBS plasmid filed under N° I-1371 at the CNCM on October 21, 1993.

## Patentansprüche

1. Vakzin, umfassend:
- einen Nukleotidvektor, wobei der Nukleotidvektor umfasst:
(i) ein Gen oder eine komplementäre DNA, die für wenigstens einen Teil des HBs-Proteins des Virus von Hepatitis B codiert,
(ii) die nicht-translatierte Terminationssequenz des Gens oder der komplementären DNA, umfassend das Polyadenylierungssignal, die sich stromabwärts des Gens oder der komplementären DNA befindet,
(iii) einen Promotor, der die Expression des genannten Gens oder der genannten komplementären DNA in Muskelzellen steuert, wobei der Promotor ausgewählt ist aus:
- dem Promotor des Cytomegalovirus,
- dem Promotor der Oberflächengene des HBV-Virus,
- einem Promotor eines Gens eines Proteins des Cytoskeletts, und
- Adjuvanzien, die kompatibel und pharmazeutisch verträglich sind.

2. Vakzin nach Anspruch 1, **dadurch gekennzeichnet, dass** der Nukleotidvektor in den Zellen nicht repliziert.

3. Vakzin nach einem der Ansprüche 1 und 2, **dadurch gekennzeichnet, dass** das Gen oder die komplementäre DNA für das Protein S, S-preS2 oder S-preS2-preS1 codiert.

4. Vakzin nach einem der Ansprüche 1 bis 3, **dadurch gekennzeichnet, dass** das Gen das Gen S ist.

5. Vakzin nach einem der Ansprüche 1 bis 4, **dadurch gekennzeichnet, dass** der Nukleotidvektor das Plasmid pCMV-HBS ist, das am 21. Oktober 1993 bei der CNCM unter der Nummer 1-1370 hinterlegt wurde.

6. Vakzin nach einem der Ansprüche 1 bis 4, **dadurch gekennzeichnet, dass** der Nukleotidvektor das Plasmid pCMVHB-S1.S2.S ist, das bei der CNCM unter der Nummer 1-1411 hinterlegt wurde.

7. Vakzin nach einem der Ansprüche 1 bis 4, **dadurch gekennzeichnet, dass** der Nukleotidvektor das Plasmid pCMVHB-S2.S ist, das bei der CNCM unter der Nummer 1-1410 hinterlegt wurde.

8. Vakzin, umfassend als Nukleotidvektor das Plasmid pRSV-HBS, das am 21. Oktober 1993 bei der CNCM unter der Nummer 1-1371 hinterlegt wurde.

9. Vakzin nach einem der Ansprüche 1 bis 4, **dadurch gekennzeichnet, dass** der Nukleotidvektor das Plasmid pHBV-S1.S2.S ist, das bei der CNCM unter der Nummber 1-1409 hinterlegt wurde.

10. Vakzin nach einem der Ansprüche 1 bis 3, **dadurch gekennzeichnet, dass** der Promotor eines Proteins des Cytoskeletts der Promotor des Desmins ist.

11. Pharmazeutische Nicht-Lipid-Zusammensetzung, die zur Immunisierung gegen eine Virusinfektion durch das Hepatitis-Virus bestimmt ist, umfassend einen Nukleotidvektor nach einem der Ansprüche 1 bis 11 und eine Substanz, die fähig ist, eine koagulatorische Nekrose der Muskelfasern zu induzieren.

12. Zusammensetzung nach Anspruch 11, **dadurch gekennzeichnet, dass** der Vektor in den Muskel des zu immunisierenden Individuums mindestens 5 Tage nach Verabreichung von Bupivacain ungefähr an denselben Ort verabreicht wird.

13. Zusammensetzung nach Anspruch 11, **dadurch gekennzeichnet, dass** der Vektor 10 Tage nach Verabreichung von Bupivacain verabreicht wird.

14. Zusammensetzung nach einem der Ansprüche 12 und 13, **dadurch gekennzeichnet, dass** die Verabreichung durch intramuskuläre Injektion durchgeführt wird.

15. Zusammensetzung nach Anspruch 13, **dadurch gekennzeichnet, dass** die intramuskuläre Injektion mit Hilfe einer Flüssigkeitsstrahlpistole durchgeführt wird.

16. Nukleotidvektor, umfassend:
(i) ein Gen oder eine komplementäre DNA, die für wenigstens einen Teil des HBs-Proteins des Virus von Hepatitis B codiert,
(ii) die nicht-translatierte Terminationssequenz des Gens oder der komplementären DNA, umfassend das Polyadenylierungssignal, die sich stromabwärts des Gens oder der komplementären DNA befindet,
(iii) einen Promotor, der die Expression des genannten Gens oder der genannten komplementären DNA in Muskelzellen steuert, wobei der Promotor ausgewählt ist aus:
- dem Promotor des Cytomegalovirus,
- dem Promotor der Oberflächengene des HBV-Virus,
- dem Promotor eines Gens eines Proteins des Cytoskeletts, und

17. Nukleotidvektor gemäß Anspruch 16, **dadurch gekennzeichnet, dass** er das Plasmid pCMV-HBS ist, das am 21. Oktober 1993 bei der CNCM unter der Nummer 1-1370 hinterlegt wurde.

18. Nukleotidvektor nach Anspruch 16, **dadurch gekennzeichnet, dass** er das Plasmid pCMVHB-S1.S2.S ist, das bei der CNCM unter der Nummer 1-1411 hinterlegt wurde.

19. Nukleotidvektor nach Anspruch 16, **dadurch gekennzeichnet, dass** er das Plasmid pCMVHB-S2.S ist, das bei der CNCM unter der Nummer 1-1410 hinterlegt wurde.

20. Nukleotidvektor nach Anspruch 16, **dadurch gekennzeichnet, dass** er das Plasmid pHBV-S1.S2.S ist, das bei der CNCM unter der Nummer 1-1409 hinterlegt wurde.

21. Nukleotidvektor, der das Plasmid pRSV-HBS ist, das am 21. Oktober 1993 bei der CNCM unter der Nummer 1-1371 hinterlegt wurde.


### LISTE DE SEQUENCES

(1) INFORMATION GENERALE:
   (i) DEPOSANT:
      (A) NOM: INSTITUT PASTEUR
      (B) RUE: 28, rue du Docteur Roux
      (C) VILLE: PARIS
      (E) PAYS: FRANCE
      (F) CODE POSTAL: 75724
   (ii) TITRE DE L' INVENTION: Vecteur nucleotidique, composition le contenant et vaccin pour l'immunisation a l'encontre d'une hepatite
   (iii) NOMBRE DE SEQUENCES: 1
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 5618 paires de bases
      (B) TYPE: acide nucléique
      (C) NOMBRE DE BRINS: double
      (D) CONFIGURATION: circulaire
   (ii) TYPE DE MOLECULE: ADN (génomique)
   (ili) HYPOTHETIQUE: NON
   (vi) ORIGINE:
      (B) SOUCHE: pRCCMV-HBS
   (xi) DESCRIPTION DE LA SEQUENCE: SEQ ID NO: 1:
   GACGGATCGG GAGATCTCCC GATCCCCTAT GGTCGACTCT    40
   CAGTACAATC TGCTCTGATG CCGCATAGTT AAGCCAGTAT    80
   CTGCTCCCTG CTTGTGTGTT GGAGGTCGCT GAGTAGTGCG   120
   CGAGCAAAAT TTAAGCTACA ACAAGGCAAG GCTTGACCGA   160
   CAATTGCATG AAGAATCTGC TTAGGGTTAG GCGTTTTGCG   200
   CTGCTTCGCG ATGTACGGGC CAGATATACG CGTTGACATT   240
   GATTATTGAC TAGTTATTAA TAGTAATCAA TTACGGGGTC 280
   ATTAGTTCAT AGCCCATATA TGGAGTTCCG CGTTACATAA 320
   CTTACGGTAA ATGGCCCGCC TGGCTGACCG CCCAACGACC 360
   CCCGCCCATT GACGTCAATA ATGACGTATG TTCCCATAGT 400
   AACGCCAATA GGGACTTTCC ATTGACGTCA ATGGGTGGAC 440
   TATTTACGGT AAACTGCCCA CTTGGCAGTA CATCAAGTGT 480
   ATCATATGCC AAGTACGCCC CCTATTGACG TCAATGACGG 520
   TAAATGGCCC GCCTGGCATT ATGCCCAGTA CATGACCTTA 560
   TGGGACTTTC CTACTTGGCA GTACATCTAC GTATTAGTCA 600
   TCGCTATTAC CATGGTGATG CGGTTTTGGC AGTACATCAA 640
   TGGGCGTGGA TAGCGGTTTG ACTCACGGGG ATTTCCAAGT 680
   CTCCACCCCA TTGACGTCAA TGGGAGTTTG TTTTGGCACC 720
   AAAATCAACG GGACTTTCCA AAATGTCGTA ACAACTCCGC 760
   CCCATTGACG CAAATGGGCG GTAGGCGTGT ACGGTGGGAG 800
   GTCTATATAA GCAGAGCTCT CTGGCTAACT AGAGAACCCA 840
   CTGCTTAACT GGCTTATCGA AATTAATACG ACTCACTATA 880
   GGGAGACCCA AGCTTGGTAC CGGGCCCCCC CTCGAGGATT 920
   GGGGACCCTG CGCTGAACAT GGAGAACATC ACATCAGGAT 960
   TCCTAGGACC CCTTCTCGTG TTACAGGCGG GGTTTTTCTT 1000
   GTTGACAAGA ATCCTCACAA TACCGCAGAG TCTAGACTCG 1040
   TGGTGGACTT CTCTCAATTT TCTAGGGGGA ACTACCGTGT 1080
   GTCTTGGCCA AAATTCGCAG TCCCCAACCT CCAATCACTC 1120
   ACCAACCTCT TGTCCTCCAA CTTGTCCTGG TTATCGCTGG 1160
   ATGTGTCTGC GGCGTTTTAT CATCTTCCTC TTCATCCTGC 1200
   TGCTATGCCT CATCTTCTTG TTGGTTCTTC TGGACTATCA 1240
   AGGTATGTTG CCCGTTTGTC CTCTAATTCC AGGATCCTCA 1280
   ACAACCAGCA CGGGACCATG CCGGACCTGC ATGACTACTG 1320
   CTCAAGGAAC CTCTATGTAT CCCTCCTGTT GCTGTACCAA 1360
   ACCTTCGGAC GGAAATTGCA CCTGTATTCC CATCCCATCA 1400
   TCCTGGGCTT TCGGAAAATT CCTATGGGAG TGGGCCTCAG 1440
   CCCGTTTCTC CTGGCTCAGT TTACTAGTGC CATTTGTTCA 1480
   GTGGTTCGTA GGGCTTTCCC CCACTGTTTG GCTTTCAGTT 1520
   ATATGGATGA TGTGGTATTG GGGGCCAAGT CTGTACAGCA 1560
   TCTTGAGTCC CTTTTTACCG CTGTTACCAA TTTTCTTTTG 1600
   TCTTTGGGTA TACATTTAAA CCCTAACAAA ACAAAGAGAT 1640
   GGGGTTACTC TCTAAATTTT ATGGGTTATG TCATTGGATG 1680
   TTATGGGTCC TTGCCACAAG AACACATCAT ACAAAAAATC 1720
   AAAGAATGTT TTAGAAAACT TCCTATTAAC AGGCCTATTG 1760
   ATTGGAAAGT ATGTCAACGA ATTGTGGGTC TTTTGGGTTT 1800
   TGCTGCCCCT TTTACACAAT GTGGTTATCC TGCGTTGATG 1840
   CCTTTGTATG CATGTATTCA ATCTAAGCAG GCTTTCACTT 1880
   TCTCGCCAAC TTACAAGGCC TTTCTGTGTA AACAATACCT 1920
   GAACCTTTAC CCCGTTGCCC GGCAACGGCC AGGTCTGTGC 1960
   CAAGTGTTTG CTGACGCAAC CCCCACTGGC TGGGGCTTGG 2000
   TCATGGGCCA TCAGCGCATG CGTGGAACCT TTTCGGCTCC 2040
   TCTGCCGATC CATACTGCGG AACTCCTAGC CGCTTGTTTT 2080
   GCTCGCAGCA GGTCTGGAGC AAACATTATC GGGACTGATA 2120
   ACTCTGTTGT CCTATCCCGC AAATATACAT CGTTTCCATG 2160
   GCTGCTAGGC TGTGCTGCCA ACTGGATCCT GCGCGGGACG 2200
   TCCTTTGTTT ACGTCCCGTC GGCGCTGAAT CCTGCGGACG 2240
   ACCCTTCTCG GGGTCGCTTG GGACTCTCTC GTCCCCTTCT 2280
   CCGTCTGCCG TTCCGACCGA CCACGGGGCG CACCTCTCTT 2320
   TACGCGGACT CCCCGTCTGT GCCTTCTCAT CTGCCGGACC 2360
   GTGTGCACTT CGCTTCACCT CTGCACGTCG CATGGAGACC 2400
   ACCGTGAACG CCCACCAAAT ATTGCCCAAG GTCTTACATA 2440
   AGAGGACTCT TGGACTCTCA GCAATGTCAA CGACCGACCT 2480
   TGAGGCATAC TTCAAAGACT GTTTGTTTAA AGACTGGGAG 2520
   GAGTTGGGGG AGGAGATTAG GTTAAAGGTC TTTGTACTAG 2560
   GAGGCTGTAG GCATAAATTG GTCTGCGCAC CAGCACCATG 2600
   CAACTTTTTC ACCTCTGCCT AATCATCTCT TGTTCATGTC 2640
   CTACTGTTCA AGCCTCCAAG CTGTGCCTTG GGTGGCTTTG 2680
   GGGCATGGAC ATCGACCCTT ATAAAGAATT TGGAGCTACT 2720
   GTGGAGTTAC TCTCGTTTTT GCCTTCTGAC TTCTTTCCTT 2760
   CAGTACGAGA TCTGGCCAGG ATCCACTAGT TCTAGAGCGG 2800
   CCGCCACCGC GGTGGAGCTC CAGCTTTTGT TCCCTTTAGT 2840
   GAGGGTTAAT TGCGCGCATG CCCGACGGCG AGGATCTCGT 2880
   CGTGACCCAT GGCGATGCCT GCTTGCCGAA TATCATGGTG 2920
   GAAAATGGCC GCTTTTCTGG ATTCATCGAC TGTGGCCGGC 2960
   TGGGTGTGGC GGACCGCTAT CAGGACATAG CGTTGGCTAC 3000
   CCGTGATATT GCTGAAGAGC TTGGCGGCGA ATGGGCTGAC 3040
   CGCTTCCTCG TGCTTTACGG TATCGCCGCT CCCGATTCGC 3080
   AGCGCATCGC CTTCTATCGC CTTCTTGACG AGTTCTTCTG 3120
   AGCGGGACTC TGGGGTTCGA AATGACCGAC CAAGCGACGC 3160
   CCAACCTGCC ATCACGAGAT TTCGATTCCA CCGCCGCCTT 3200
   CTATGAAAGG TTGGGCTTCG GAATCGTTTT CCGGGACGCC 3240
   GGCTGGATGA TCCTCCAGCG CGGGGATCTC ATGCTGGAGT 3280
   TCTTCGCCCA CCCCAACTTG TTTATTGCAG CTTATAATGG 3320
   TTACAAATAA AGCAATAGCA TCACAAATTT CACAAATAAA 3360
   GCATTTTTTT CACTGCATTC TAGTTGTGGT TTGTCCAAAC 3400
   TCATCAATGT ATCTTATCAT GTCTGGATCC CGTCGACCTC 3440
   GAGAGCTTGG CGTAATCATG GTCATAGCTG TTTCCTGTGT 3480
   GAAATTGTTA TCCGCTCACA ATTCCACACA ACATACGAGC 3520
   CGGAAGCATA AAGTGTAAAG CCTGGGGTGC CTAATGAGTG 3560
   AGCTAACTCA CATTAATTGC GTTGCGCTCA CTGCCCGCTT 3600
   TCCAGTCGGG AAACCTGTCG TGCCAGCTGC ATTAATGAAT 3640
   CGGCCAACGC GCGGGGAGAG GCGGTTTGCG TATTGGGCGC 3680
   TCTTCCGCTT CCTCGCTCAC TGACTCGCTG CGCTCGGTCG 3720
   TTCGGCTGCG GCGAGCGGTA TCAGCTCACT CAAAGGCGGT 3760
   AATACGGTTA TCCACAGAAT CAGGGGATAA CGCAGGAAAG 3800
   AACATGTGAG CAAAAGGCCA GCAAAAGGCC AGGAACCGTA 3840
   AAAAGGCCGC GTTGCTGGCG TTTTTCCATA GGCTCCGCCC 3880
   CCCTGACGAG CATCACAAAA ATCGACGCTC AAGTCAGAGG 3920
   TGGCGAAACC CGACAGGACT ATAAAGATAC CAGGCGTTTC 3960
   CCCCTGGAAG CTCCCTCGTG CGCTCTCCTG TTCCGACCCT 4000
   GCCGCTTACC GGATACCTGT CCGCCTTTCT CCCTTCGGGA 4040
   AGCGTGGCGC TTTCTCAATG CTCACGCTGT AGGTATCTCA 4080
   GTTCGGTGTA GGTCGTTCGC TCCAAGCTGG GCTGTGTGCA 4120
   CGAACCCCCC GTTCAGCCCG ACCGCTGCGC CTTATCCGGT 4160
   AACTATCGTC TTGAGTCCAA CCCGGTAAGA CACGACTTAT 4200
   CGCCACTGGC AGCAGCCACT GGTAACAGGA TTAGCAGAGC 4240
   GAGGTATGTA GGCGGTGCTA CAGAGTTCTT GAAGTGGTGG 4280
   CCTAACTACG GCTACACTAG AAGGACAGTA TTTGGTATCT 4320
   GCGCTCTGCT GAAGCCAGTT ACCTTCGGAA AAAGAGTTGG 4360
   TAGCTCTTGA TCCGGCAAAC AAACCACCGC TGGTAGCGGT 4400
   GGTTTTTTTG TTTGCAAGCA GCAGATTACG CGCAGAAAAA 4440
   AAGGATCTCA AGAAGATCCT TTGATCTTTT CTACGGGGTC 4480
   TGACGCTCAG TGGAACGAAA ACTCACGTTA AGGGATTTTG 4520
   GTCATGAGAT TATCAAAAAG GATCTTCACC TAGATCCTTT 4560
   TAAATTAAAA ATGAAGTTTT AAATCAATCT AAAGTATATA 4600
   TGAGTAAACT TGGTCTGACA GTTACCAATG CTTAATCAGT 4640
   GAGGCACCTA TCTCAGCGAT CTGTCTATTT CGTTCATCCA 4680
   TAGTTGCCTG ACTCCCCGTC GTGTAGATAA CTACGATACG 4720
   GGAGGGCTTA CCATCTGGCC CCAGTGCTGC AATGATACCG 4760
   CGAGACCCAC GCTCACCGGC TCCAGATTTA TCAGCAATAA 4800
   ACCAGCCAGC CGGAAGGGCC GAGCGCAGAA GTGGTCCTGC 4840
   AACTTTATCC GCCTCCATCC AGTCTATTAA TTGTTGCCGG 4880
   GAAGCTAGAG TAAGTAGTTC GCCAGTTAAT AGTTTGCGCA 4920
   ACGTTGTTGC CATTGCTACA GGCATCGTGG TGTCACGCTC 4960
   GTCGTTTGGT ATGGCTTCAT TCAGCTCCGG TTCCCAACGA 5000
   TCAAGGCGAG TTACATGATC CCCCATGTTG TGCAAAAAAG 5040
   CGGTTAGCTC CTTCGGTCCT CCGATCGTTG TCAGAAGTAA 5080
   GTTGGCCGCA GTGTTATCAC TCATGGTTAT GGCAGCACTG 5120
   CATAATTCTC TTACTGTCAT GCCATCCGTA AGATGCTTTT 5160
   CTGTGACTGG TGAGTACTCA ACCAAGTCAT TCTGAGAATA 5200
   GTGTATGCGG CGACCGAGTT GCTCTTGCCC GGCGTCAATA 5240
   CGGGATAATA CCGCGCCACA TAGCAGAACT TTAAAAGTGC 5280
   TCATCATTGG AAAACGTTCT TCGGGGCGAA AACTCTCAAG 5320
   GATCTTACCG CTGTTGAGAT CCAGTTCGAT GTAACCCACT 5360
   CGTGCACCCA ACTGATCTTC AGCATCTTTT ACTTTCACCA 5400
   GCGTTTCTGG GTGAGCAAAA ACAGGAAGGC AAAATGCCGC 5440
   AAAAAAGGGA ATAAGGGCGA CACGGAAATG TTGAATACTC 5480
   ATACTCTTCC TTTTTCAATA TTATTGAAGC ATTTATCAGG 5520
   GTTATTGTCT CATGAGCGGA TACATATTTG AATGTATTTA 5560
   GAAAAATAAA CAAATAGGGG TTCCGCGCAC ATTTCCCCGA 5600
   AAAGTGCCAC CTGACGTC 5618